(19) Europäisches Patentamt
European Patent Office
Office européen des brevets

(11) **EP 1 124 186 B1**

(12) **EUROPÄISCHE PATENTSCHRIFT**

(45) Veröffentlichungstag und Bekanntmachung des Hinweises auf die Patenterteilung:
**11.01.2012 Patentblatt 2012/02**

(51) Int Cl.:
*G06F 17/00* (2006.01)       *A61B 5/0245* (2006.01)
*G01R 23/16* (2006.01)       *A61N 1/365* (2006.01)

(21) Anmeldenummer: **01101489.1**

(22) Anmeldetag: **24.01.2001**

(54) **Verfahren zur Berechnung der Herzratenvariabilität zur Anwendung in einem EKG-Monitor sowie EKG-Monitor mit einem entsprechenden Berechnungsprogramm**

Method for computing the heartrate variability for use in an ECG-monitor and an ECG-monitor comprising such a computational program

Méthode de calcul de la variabilité du rythme cardiaque pour utilisation dans un moniteur ECG et un moniteur ECG comprenant un programme de calcul correspondant

(84) Benannte Vertragsstaaten:
**AT BE CH CY DE DK ES FI FR GB GR IE IT LI LU MC NL PT SE TR**

(30) Priorität: **11.02.2000   DE 10006154**

(43) Veröffentlichungstag der Anmeldung:
**16.08.2001   Patentblatt 2001/33**

(73) Patentinhaber: **BIOTRONIK SE & Co. KG**
**12359 Berlin (DE)**

(72) Erfinder:
• **Meier, Jan H., Dr.**
  **91080 Uttenreuth (DE)**
• **Fournié, Eric**
  **91052 Erlangen (DE)**

(74) Vertreter: **Lindner-Vogt, Karin L. et al**
**Biotronik SE & Co. KG**
**Woermannkehre 1**
**12359 Berlin (DE)**

(56) Entgegenhaltungen:
WO-A-86/02250       US-A- 5 749 367
US-A- 5 827 195       US-A- 5 967 995

• **LOVETT E G ET AL: "Approximate minimum bias multichannel spectral estimation for heart rate variability" ANNALS OF BIOMEDICAL ENGINEERING BLACKWELLS SCIENTIFIC PUBLICATIONS USA, Bd. 25, Nr. 3, 1997, Seiten 509-520, XP002551738 ISSN: 0090-6964**

**Beschreibung**

Die Erfindung betrifft ein Verfahren zur Berechnung der Herzratenvariabilität des menschlichen Herzens zur Anwendung in einem EKG-Monitor sowie einen EKG-Monitor, dessen Betriebsprogramm ein solches Berechnungsverfahren implementiert. Unter "EKG-Monitor" soll dabei jedwedes externes oder implantierbares Gerät verstanden werden, das EKG-Signale detektiert und auswertet, also neben den eigentlichen Monitoren z. B. auch die entsprechenden funktionalen Komponenten in Herzschrittmachern und Defibrillatoren.

Zum Hintergrund der Erfindung ist auszuführen, daß die Herzratenvariabilität ein in der klinischen Praxis etablierter Risikoindikator bei Post-Infarkt-Patienten ist. Es ist daher ein erklärtes Ziel der Kardiologie, die Herzratenvariabilität bei solchen Post-Infarkt-Patienten aufzuzeichnen und auszuwerten, um im Falle alarmierender Werte geeignete Schritte einleiten zu können.

Ein anderer Aspekt im Zusammenhang mit der vorliegenden Erfindung ist die herrschende Tendenz, kardiologische Überwachungsgeräte für herzkranke Patienten so zu konzipieren, daß das Gerät in den Körper des Patienten implantierbar ist.

Bei der Bestimmung der Herzratenvariabilität wird nun das Frequenzspektrum der Herzrate bestimmt und die Verhältnisse der auftretenden Maxima ausgewertet. Das Frequenzspektrum wird dabei üblicherweise mit Hilfe der sogenannten "Fourier-Transformation" analysiert, indem das vom EKG-Monitor empfangene EKG-Signal während eines bestimmten Abtastintervalls abgetastet und daraus eine Reihe diskreter, für die Herzratenvariabilität repräsentativer Meßwerte, wie z. B. die RR-Intervalle, ermittelt werden. Ein solches Verfahren ist z. B, in der Patentschrift US 5,967,995 offenbart.

Wie im Zuge der Beschreibung des Ausführungsbeispiels unter Bezugnahme auf entsprechende Formeln noch detaillierter dargelegt wird, wird das Frequenzspektrum der Meßwerte aus den Fourier-Koeffizienten der Fourier-Transformation berechnet. Die Fourier-Koeffizienten wiederum werden aus einer Kombination der Meßwerte mit den sinus- und kosinusförmigen Fourier-Vektoren der Fourier-Transformation berechnet.

Problematisch bei der üblichen Fourier-Transformation ist die Tatsache, daß zur rechnerischen Implementierung die (kontinuierlichen) Fourier-Vektoren in diskrete Stützstellen übergeführt werden müssen. Die Stützstellen sind reelle Zahlen, die den sinus- und kosinusförmigen Verlauf der Fourier-Vektoren wiedergeben. In Abhängigkeit von der gewählten Anzahl von Stützstellen steigt nun die Anzahl der Multiplikationen, die zum Errechnen der Fourier-Koeffizienten aus den Fourier-Vektoren und den Meßwerten erforderlich sind, übeipropoitional an. Entsprechend erfordert die hohe Anzahl an Multiplikationsvorgängen mit reellen Zahlen einen extremen Rechenaufwand und einen enorm hohen Bedarf an Speicherkapazität. Ein Beispiel für die Fourier-Transfonnation ist in der Patenschrift WO 86/02250 veröffentlicht worden.

Der hohe Rechen- und Speicheraufwand übersteigt die Kapazität von für kardiologische Geräteimplantate verfügbaren Mikroprozessorsystemen und deren Energieversorgung bei weitem, so daß die Anwendung einer Fourier-Transformation zur Frequenzanalyse mit den derzeit gängigen Berechnungsverfahren und Prozessorsystemen nicht möglich ist.

Zur Lösung dieser Problematik schlägt nun die Erfindung vor, die bekannte (Fast-)Fourier-Transformation bei der Analyse der Herzratenvariabilität durch ein Näherungsverfahren zu ersetzen, das auf der Basis der Fourier-Transformation die Vektor-Zahlenwerte für die Berechnung des Frequenzspektrum der abgetasteten Meßwerte durch Vektor-Näherungswerte einer begrenzten Anzahl ersetzt, wobei die Vektor-Näherungswerte den Verlauf der Fourier-Vektoren nachbilden. Durch diese Maßnahme wird eine deutlich Verringerung des Rechen- und Speicher-Aufwandes erzielt, was die Anwendung in Geräte-Implantaten ermöglicht. Die Vereinfachung wird dadurch erreicht, daß die Fourier-Vektoren nicht mehr durch Stützstellen der Sinus- und Kosinusfunktionen in Form reeller Zahlen dargestellt werden. Statt dessen werden die Stützstellen als, ganzzahlige Vektor-Näherungsweite auf der Basis der Zahlen -1, 0 und +1 gewählt. Zusammenfassend wird durch diese "Vergröberung" der Stützstellen eine Art "Fuzzy-Fourier-Transformation" geschaffen.

Günstige Auswerteergebnisse haben sich ergeben, wenn die Umsetzung der reellen Vektor-Zahlenwerte in die Näherungswerte -1, 0 und +1 mit einer Grenze von +/-0,33 erfolgt. Für einen Vektorzahlenwert zwischen -0,33 und +0,33 wird der Näherungswert zu 0 gesetzt, für Vektorzahlenwerte > +0,33 bzw. < -0,33 werden die Näherungswerte zu +1 bzw. -1 gesetzt.

Da bei der herkömmlichen Fourier-Transformation die Summe der Vektor-Näherungswerte für jeden Fourier-Vektor einen Wert von 0 ergibt, kann bei einer Abweichung der Summe der Vektor-Näherungswerte vom Wert 0 mindestens ein Näherungswert so korrigiert werden, daß der Summenwert 0 beträgt. Damit wird ein scheinbarer Offset in dem analysierten Frequenzspektrum bzw. ein falscher Peak im berechneten Frequenzspektrum bei niedrigen Frequenzen vermieden.

Die Erfindung bezieht sich ferner auf einen EKG-Monitor mit einem Betriebsprogramm, das das vorstehend erörterte Berechnungsverfahren implementiert. Dadurch wird der EKG-Monitor in den menschlichen Körper implantierbar bzw. kann in einen Herzschrittmacher integriert werden.

Im folgenden wird das erfindungsgemäße Berechnungsverfahren anhand eines Ausführungsbeispiels unter Bezugnahme auf die beigefügten Zeichnungen näher erläutert. Es zeigen:

Fig. 1 ein Vergleichsdiagramm des Sinus-Eigenvektors der Fourier- Transformation und dessen Nachbildung durch ganzzahlige Vektor- Näherungswerte,

Fig. 2 eine Darstellung der mittels des Fuzzy-Fourier-Transformations- Näherungsverfahrens berechneten Spektralkomponenten eines ein- zelnen Sinussignals,

Fig. 3 eine Darstellung der entsprechend berechneten Spektralkomponen- ten und frequenzabhängigen Amplituden für eine Mischung von zwei Sinussignalen und einem Rauschsignal,

Fig. 4 Diagramme eines RR-Intervallverlaufs aus einem EKG-Abschnitt und der mit dem Fuzzy-Fourier-Transformations-Näherungs- verfahren berechneten Spektralkomponenten sowie Frequenzanaly- se, und

Fig. 5 ein Diagramm analog Fig. 1 mit korrigierten Vektor- Näherungswerten.

Zum besseren Verständnis der Erfindung wird eingangs dieser Beschreibung kurz das Berechnungsprinzip der Fourier-Transformation rekapituliert. So besagen die Fourierschen Theoreme, daß sich eine Serie von Meßwerten $R_i$ (i=0 ... 2N-1) beschreiben läßt als

$$R_i = 1/N \sum_{n=1}^{N-1} s_n \sin(\pi n i/N) + 1/N \sum_{m=0}^{N-1} c_m \cos(\pi m i/N) \qquad (1)$$

Die Spektralkomponenten $s_n$ und $c_n$ sind definiert durch

$$s_n = \sum_{i=0}^{2N-1} R_i \sin(\pi n i/N) = \overline{R} \bullet \overline{\sin}_n$$

$$c_0 = \tfrac{1}{2} \sum_{j=0}^{2N-1} s_i \qquad\qquad (2)$$

$$c_m = \sum_{j=0}^{2N-1} R_i \cos(\pi m j/N) = \overline{R} \bullet \overline{\cos}_n$$

Die Fourier-Koeffizienten ergeben sich also aus einer Linearkombination des Meßwertvektors $\overline{R}$ und der sinus- bzw. kosinusförmigen Fourier-Vektoren $\overline{\sin}_n$ bzw. $\overline{\cos}_n$. Diese Fourier-Vektoren

$$\overline{\sin}_n = (0, \sin(\pi n/N), \sin(2\pi n/N), \ldots, \sin((2N-1)\pi n/N)) \qquad (3)$$

$$\cos_n = (0, \cos(\pi n/N), \cos(2\pi n/N), \ldots, \cos((2N-1)\pi n/N))$$

sind die Eigenvektoren des Systems.

Für die Amplitude A einer Frequenz-Komponente f gilt nun bei einem Abtastintervall von $\Delta t$:

$$A\ (f = n\ /\ 2N\Delta t) = \sqrt{(s_n{}^2 + c_n{}^2)} \qquad\qquad (4)$$

Würde das vorstehende Gleichungssystem durch ein Steuerprogramm in einem EKG-Monitor implementiert werden, so wären bei einem Wert von N=16 im Gleichungssystem (2) für diese diskrete Fourier-Transformation bereits 1024 Multiplikationen erforderlich. Für die Eigenvektoren bei 8-Bit-Auflösung und 32 Eigenvektoren mit 32 Komponenten würde sich ein Speicherbedarf von 1 kByte ergeben. Diese Anforderungen machen eine Realisierung im Implantat unmöglich.

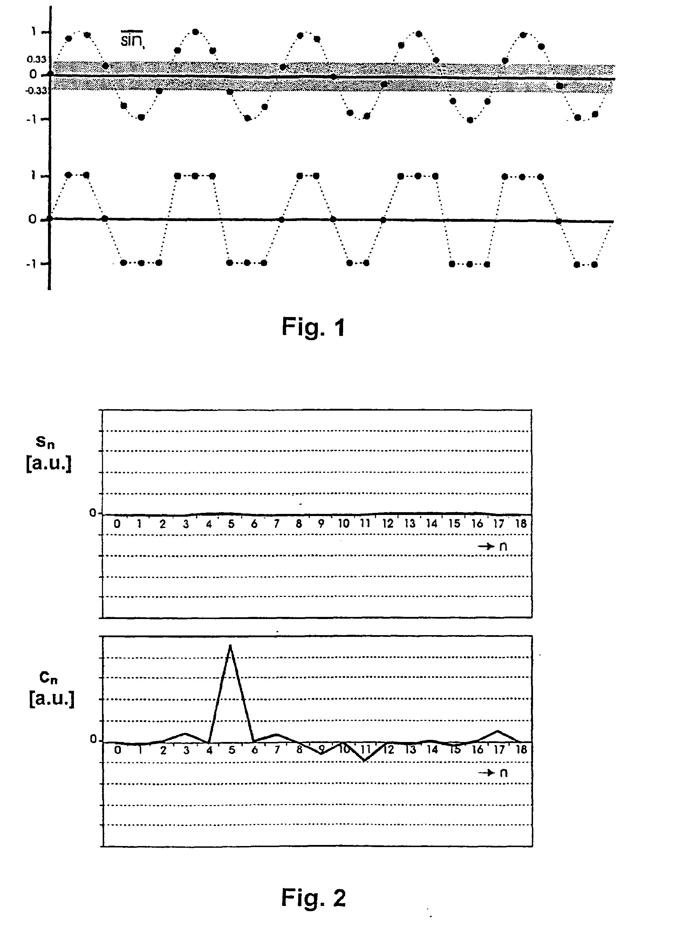

Die erfindungsgemäße Fuzzy-Fourier-Transformation ist nun anhand von Fig. 1 zu erläutern. Demnach werden die Vektor-Zahlenweite für die Fourier-Vektoren gemäß den Formeln (3) durch ganzzahlige Vektor-Näherungswerte, nämlich die drei Werte -1, 0 und +1 ersetzt. Die Zuordnungsvorschrift lautet wie folgt:

- -0,33 < Vektor-Zahlenwert < +0,33:   Näherungswert = 0
- Vektor-Zahlenwert < -0,33:   Näherungswert = -1
- Vektor-Zahlenwert > +0,33:   Näherungswert = +1

Fig. 1 zeigt diese Näherung für die Größen 2N=32, n=4. Im oberen Bereich ist der Sinus-Eigenvektor mit entsprechenden Stützstellen in Form reeller Zahlenwerte dargestellt. Nach der obigen Zuordnungsvorschrift wird dieser Sinus-Eigenvektor in den in der unteren Darstellung von Fig. 1 gezeigten Verlauf übergeführt. Es werden also die Vektor-Zahlenwerte $\sin(\pi ni/N)$ und $\cos(\pi ni/N)$ in den Gleichungen (2) ersetzt durch -1, 0 bzw. 1. Damit sind zur Berechnung der Größen $s_n$ und $c_n$ keine Multiplikationen mehr nötig, sondern im wesentlichen nur noch Additionen erforderlich. Zudem können die Komponenten der Eigenvektoren in Form der Vektor-Näherungswerte jeweils in Speichern mit 2-Bit-Auflösung abgespeichert werden, was den benötigen Speicheraufwand gegenüber dem oben erörterten Beispiel um einen Faktor 4 reduziert.

Die oben erwähnten genäherten Vektor-Zahlenwerte für die Fuzzy-Fourier-Transformation können einerseits in einer Tabelle abgespeichert werden, die diese trigonometrischen Funktionsnäherungen beschreibt. Alternativ dazu können auch die Punkte mit geringem Rechenaufwand berechnet werden, bei denen auf der Basis der oben bezeichneten Zuordnungsvorschrift die Werte der Funktionen geändert werden. Es findet als eine Art "Online-Berechnung" der Fuzzy-Fourier-Vektorstützstellen statt, was Einsparungen an Rechenkapazität und Energiebedarf gegenüber der Speicherung vorberechneter Stützstellen bringt.

So gilt für den Näherungswert = +1 folgende Gleichung:

$$\sin\ (\pi ni/N) \geq 0,33$$

$$\Rightarrow 0,3363 \ +/-\ 2\pi \leq \pi ni/N \leq 2,8053 \ +/-\ 2\pi$$

$$\Rightarrow 0,1070\ N \ +/-\ 2N \leq \pi ni \leq 0,8930\ N \ +/-\ 2N$$

Der Näherungswert 0 ergibt sich für

$$0,8930\ N \ +/-\ 2N < ni < 1,1070\ N \ +/-\ 2N$$

Der Näherungswert -1 ergibt sich für

$$1,070\ N \ +/-\ 2N \leq ni \leq 1,8930\ N \ +/-\ 2N$$

Analog ergibt sich die Berechnung der Kosinus-Näherungswerte zum

- Näherungswert = +1, wenn ni > 0 +/- 2N und < als 0,393 N +/- 2N oder > 1,6070 N +/- 2N und < 2N +/- 2N
- Näherungswert -1, wenn ni zwischen 0,6070 N +/- 2N und 1,3930 N +/- 2N liegt sowie
- Näherungswert 0, wenn ni außerhalb der oben stehenden Wertebereiche liegt.

Als weitere Vereinfachung läßt sich schließlich die Gleichung (4) ersetzen durch

$$A\ (f = n\ /\ 2N\Delta t) \approx |s_n| + |c_n| \qquad\qquad (5)$$

In einem Simulationsversuch wurde die vorstehend in ihrer Basis dargestellte Fuzzy-Fourier-Transformation angewendet und entsprechende Spektralkomponenten $s_n$ und $c_n$ berechnet und mit der diskreten Fourier-Transformation mit exakten Werten verglichen. Als Eingangssignal $R_i$ wurde dabei folgendes Signal herangezogen, bei dem es sich um eine Summe von zwei Sinussignalen, einem Rauschsignal ("Random") und einem Offset handelt:

$$R_i = C_1\ \sin(\delta\varphi_1 i + \varphi'_1) + C_2\ \sin(\delta\varphi_2 i + \varphi'_2) + C_3\ Random_i[-1,...+1] + C_4$$

Die Amplitudenkoëffizienten $C_1$, $C_2$, $C_3$, $C_4$ und die Phasenkoeffizienten $\delta\varphi_1$, $\varphi'_1$, $\delta\varphi_2$ und $\varphi'_2$ sind frei wählbar.

Als Simulationsversuch wurde ein einzelnes Sinussignal mit $\delta\varphi_1 = 5\pi/19$ und $\varphi_1' = \pi/4$ berechnet. Die Amplitudenkoeffizienten $C_2$, $C_3$ und $C_4$ wurden zu 0 gesetzt.

Die diskrete Fourier-Transformation ergibt nur einen einzelnen diskreten Peak im $c_n$-Verlauf bei n=5, da die Phasenverschiebung von $\pi/4$ ein Kosinussignal zugrundelegt. In Fig. 2 ist dieser Peak nicht eigens dargestellt.

Eine Anwendung des Fuzzy-Fourier-Transfoimations-Nähemngsverfahrens ergibt die in Fig. 2 gezeigten Verläufe der Spektralkomponenten $s_n$ und $c_n$. Daraus ist unmittelbar erkennbar, daß auch das Näherungsverfahren den Peak bei n=5 in der Spektralkomponente $c_n$ reproduziert, was für die Richtigkeit des Näherungsverfahrens ein Beleg ist. Allerdings sind die Basislinien von $s_n$ und $c_n$ nicht konstant gleich 0 und schwanken um diesen Wert. Es werden bis zu 20 % des maximalen Amplitudenwertes erreicht, was allerdings verkraftbar ist.

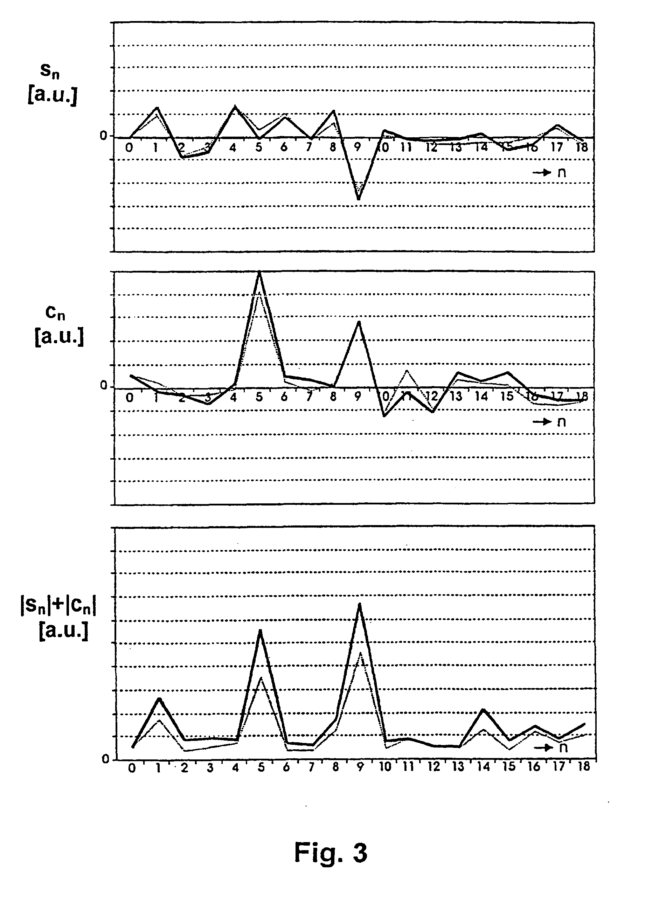

Eine komplexere Simulation mit dem oben angegebenen Eingangssignal ist im Ergebnis in Fig. 3 dargestellt. Dabei wurde eine Mischung von zwei verrauschten Sinussignalen mit folgenden Parametern zugrundegelegt:

$$C_1 = C_2 = 0{,}5\ C_3 \ ; \ \delta\varphi_1 = 5\pi/19 \ ; \ \varphi'_1 = 0 \ ; \ \delta\varphi_2 = 9\pi/19 \ ; \ \varphi'_1 = \pi/4$$

In Fig. 3 sind die beiden Spektralkomponenten $s_n$ und $c_n$ sowie die mit Hilfe der Formel (5) berechnete frequenzabhängige Amplitude A dargestellt. Die Ergebnisse mit dem erfindungsgemäßen Näherungsverfahren sind dabei mit durchgezogenen, die der diskreten Fourier-Transformation mit punktierten Linien aufgetragen.

Vergleicht man die beiden Signalverläufe in den einzelnen Diagrammteilen mit einander, so ist erkennbar, daß die Resultate der diskreten und der Fuzzy-Fourier-Transformation einander sehr ähnlich und das Näherungsverfahren damit brauchbar ist. Lediglich die Basislinie der frequenzabhängigen Amplitude A(f) im untersten Teil der Fig. 3 wurde durch den Rauschanteil angehoben. Obwohl jedoch das Rauschsignal doppelt so groß wie die zwei Sinussignale sind, ist die Existenz dieser beiden Signale im Frequenzspektrum bei n=5 und n=9 erkennbar.

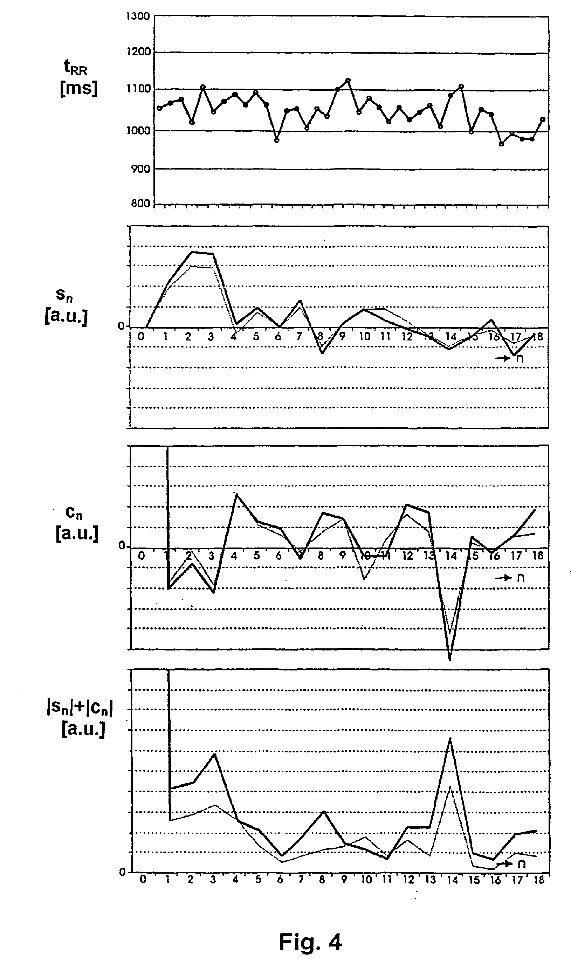

Anhand von Fig. 4 sind die Auswerteergebnisse mit Hilfe des erfindunggemäßen Näherungsverfahrens in einem konkreten praktischen Beispiel dargestellt. So zeigt das oberste Diagramm in Fig. 4 einen Ausschnitt der Messung der RR-Intervalle, wie sie aus einem EKG-Signal durch Abtastung während eines Abtastinterveralls von 1,25 sec. ermittelt wurden. Erkennbar schwanken die Periodendauern der RR-Intervalle zwischen ca. 970 und 1130 ms.

Bei Anwendung der diskreten Fourier-Transformation und des vorstehenden Näherungsverfahrens ergeben sich für beide Berechnungsarten Maxima bei n=3 und n=14, wie dem unteren Diagramm der Fig. 4 entnehmbar ist. Diese beiden Maxima liegen bei Umrechnung in den Frequenzraum bei Werten von 0,075 Hz und 0,35 Hz.

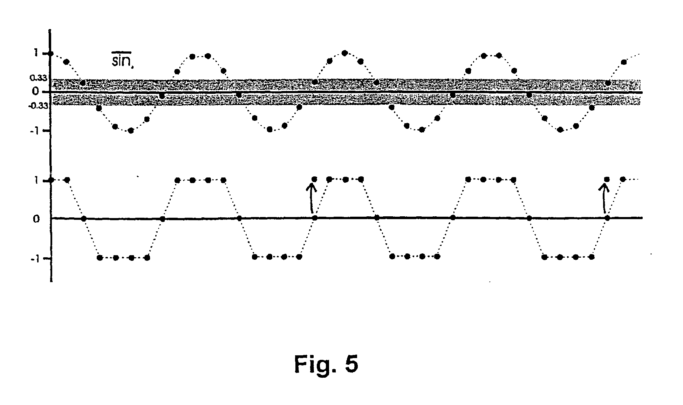

Bei Durchführung des Näherungsverfahrens wurde im übrigen festgestellt, daß die bei der diskreten Fourier-Transformation geltende Bedingung, daß die Summe der Komponenten eines einzelnen Eigenvektors (mit Ausnahme des Vektors $\overline{\cos_0}$) gleich 0 ist, bei einigen Annäherungen der Kosinus-Eigenvektoren nicht erfüllt ist. Es ergab sich eine Summe von -2 oder 2, wie dies beispielsweise bei den im unteren Diagramm von Fig. 5 durch eine strichlierte Linie unterlegten Vektor-Näherungswerten der Fall ist. Dies würde sich in einem starken niederfrequenten Peak im berechneten Frequenzspektrum äußern. Zu dessen Vermeidung werden die Vektor-Näherungswerte so korrigiert, daß der Summenwert 0 beträgt. In dem in Fig. 5 gezeigten Beispiel werden zwei Näherungswerte mit dem Wert 0 auf den Wert +1 angehoben, wie dies durch die beiden Pfeile angedeutet ist. Durch diese Korrektur beträgt die Summe der Vektor-Näherungswerte 0.

Abschließend ist darauf hinzuweisen, daß die Vektorlänge 2N einen wesentlichen Einfluß auf die Genauigkeit der Fuzzy-Fourier-Transformation hat. Läßt sich die Länge N in eine Mehrzahl kleinerer Vektoren von gleicher Länge 1 verteilen, so daß gilt N=n•1 und es treten bei der Umsetzung der Eigenvektoren gleiche Annäherungen mehrfach auf. Dies kann bei bestimmten Frequenzen deutliche Nebenmaxima bis zu 30 % im berechneten Frequenzspektrum A(f) erzeugen. Optimal ist daher eine Vektorlänge, bei der die Länge N einer Primzahl entspricht, d. h.

$$2N = 2 \cdot \text{Primzahl}.$$

In den vorstehend beschriebenen Beispielen betrug der Wert von 2N=2•19=38.

In den anhängenden Tabellen 1 und 2 sind schließlich geeignete Vektor-Näherungswerte für die Eigenvektor $\sin_{n,i}$ und $\cos_{n,i}$ aufgezeigt.

Näherungen für $\sin_{n,i}$

Tabelle 1

| i | n | | | | | | | | | | | | | | | | | | |
|---|---|---|---|---|---|---|---|---|---|---|---|---|---|---|---|---|---|---|---|
|   | 0 | 1 | 2 | 3 | 4 | 5 | 6 | 7 | 8 | 9 | 10 | 11 | 12 | 13 | 14 | 15 | 16 | 17 | 18 |
| 0 | 0 | 0 | 0 | 0 | 0 | 0 | 0 | 0 | 0 | 0 | 0 | 0 | 0 | 0 | 0 | 0 | 0 | 0 | 0 |
| 1 | 0 | 0 | 0 | 1 | 1 | 1 | 1 | 1 | 1 | 1 | 1 | 1 | 1 | 1 | 1 | 1 | 1 | 0 | 0 |
| 2 | 0 | 0 | 1 | 1 | 1 | 1 | 1 | 1 | 1 | 0 | 0 | -1 | -1 | -1 | -1 | -1 | -1 | -1 | 0 |
| 3 | 0 | 1 | 1 | 1 | 1 | 1 | 0 | 0 | -1 | -1 | -1 | -1 | 0 | 0 | 1 | 1 | 1 | 1 | 1 |
| 4 | 0 | 1 | 1 | 1 | 1 | 0 | -1 | -1 | -1 | 0 | 0 | 1 | 1 | 1 | 0 | -1 | -1 | -1 | -1 |
| 5 | 0 | 1 | 1 | 1 | 0 | -1 | -1 | -1 | 0 | 1 | 1 | 0 | -1 | -1 | -1 | 0 | 1 | 1 | 1 |
| 6 | 0 | 1 | 1 | 0 | -1 | -1 | 0 | 1 | 1 | 1 | -1 | -1 | -1 | 0 | 1 | 1 | 0 | -1 | -1 |
| 7 | 0 | 1 | 1 | 0 | -1 | -1 | 1 | 1 | 0 | -1 | -1 | 0 | 1 | 1 | -1 | -1 | 0 | 1 | 1 |
| 8 | 0 | 1 | 1 | -1 | -1 | 0 | 1 | 0 | -1 | -1 | 1 | 1 | 0 | -1 | 0 | 1 | 1 | -1 | -1 |
| 9 | 0 | 1 | 0 | -1 | 0 | 1 | 1 | -1 | -1 | 1 | 1 | -1 | -1 | 1 | 1 | 0 | -1 | 0 | 1 |
| 10 | 0 | 1 | 0 | -1 | 0 | 1 | -1 | -1 | 1 | 1 | -1 | -1 | 1 | 1 | -1 | 0 | 1 | 0 | -1 |
| 11 | 0 | 1 | -1 | -1 | 1 | 0 | -1 | 0 | 1 | -1 | -1 | 1 | 0 | -1 | 0 | 1 | -1 | -1 | 1 |
| 12 | 0 | 1 | -1 | 0 | 1 | -1 | -1 | 1 | 0 | -1 | 1 | 0 | -1 | 1 | 1 | -1 | 0 | 1 | -1 |
| 13 | 0 | 1 | -1 | 0 | 1 | -1 | 0 | 1 | -1 | 1 | 1 | -1 | 1 | 0 | -1 | 1 | 0 | -1 | 1 |
| 14 | 0 | 1 | -1 | 1 | 0 | -1 | 1 | -1 | 0 | 1 | -1 | 0 | 1 | -1 | 1 | 0 | -1 | 1 | -1 |
| 15 | 0 | 1 | -1 | 1 | -1 | 0 | 1 | -1 | 1 | 0 | 0 | 1 | -1 | 1 | 0 | -1 | 1 | -1 | 1 |
| 16 | 0 | 1 | -1 | 1 | -1 | 1 | 0 | 0 | 1 | -1 | 1 | -1 | 0 | 0 | -1 | 1 | -1 | 1 | -1 |
| 17 | 0 | 0 | -1 | 1 | -1 | 1 | -1 | 1 | -1 | 0 | 0 | -1 | 1 | -1 | 1 | -1 | 1 | -1 | 0 |
| 18 | 0 | 0 | 0 | 1 | -1 | 1 | -1 | 1 | -1 | 1 | -1 | 1 | -1 | 1 | -1 | 1 | -1 | 0 | 0 |
| 19 | 0 | 0 | 0 | 0 | 0 | 0 | 0 | 0 | 0 | 0 | 0 | 0 | 0 | 0 | 0 | 0 | 0 | 0 | 0 |
| 20 | 0 | 0 | 0 | -1 | 1 | -1 | 1 | -1 | 1 | -1 | 1 | -1 | 1 | -1 | 1 | -1 | 1 | 0 | 0 |
| 21 | 0 | 0 | 1 | -1 | 1 | -1 | 1 | -1 | 1 | 0 | 0 | 1 | -1 | 1 | -1 | 1 | -1 | 1 | 0 |
| 22 | 0 | -1 | 1 | -1 | 1 | -1 | 0 | 0 | -1 | 1 | -1 | 1 | 0 | 0 | 1 | -1 | 1 | -1 | 1 |
| 23 | 0 | -1 | 1 | -1 | 1 | 0 | -1 | 1 | -1 | 0 | 0 | -1 | 1 | -1 | 0 | 1 | -1 | 1 | -1 |
| 24 | 0 | -1 | 1 | -1 | 0 | 1 | -1 | 1 | 0 | -1 | 1 | 0 | -1 | 1 | -1 | 0 | 1 | -1 | 1 |
| 25 | 0 | -1 | 1 | 0 | -1 | 1 | 0 | -1 | 1 | -1 | -1 | 1 | -1 | 0 | 1 | -1 | 0 | 1 | -1 |
| 26 | 0 | -1 | 1 | 0 | -1 | 1 | 1 | -1 | 0 | 1 | -1 | 0 | 1 | -1 | -1 | 1 | 0 | -1 | 1 |
| 27 | 0 | -1 | 1 | 1 | -1 | 0 | 1 | 0 | -1 | 1 | 1 | -1 | 0 | 1 | 0 | -1 | 1 | 1 | -1 |
| 28 | 0 | -1 | 0 | 1 | 0 | -1 | 1 | 1 | -1 | -1 | 1 | 1 | -1 | -1 | 1 | 0 | -1 | 0 | 1 |

(fortgesetzt)

| i | n | | | | | | | | | | | | | | | | | | |
|---|---|---|---|---|---|---|---|---|---|---|---|---|---|---|---|---|---|---|---|
| | 0 | 1 | 2 | 3 | 4 | 5 | 6 | 7 | 8 | 9 | 10 | 11 | 12 | 13 | 14 | 15 | 16 | 17 | 18 |
| 29 | 0 | -1 | 0 | 1 | 0 | -1 | -1 | 1 | 1 | -1 | -1 | 1 | 1 | -1 | -1 | 0 | 1 | 0 | -1 |
| 30 | 0 | -1 | -1 | 1 | 1 | 0 | -1 | 0 | 1 | 1 | -1 | -1 | 0 | 1 | 0 | -1 | -1 | 1 | 1 |
| 31 | 0 | -1 | -1 | 0 | 1 | 1 | -1 | -1 | 0 | 1 | 1 | 0 | -1 | -1 | 1 | 1 | 0 | -1 | -1 |
| 32 | 0 | -1 | -1 | 0 | 1 | 1 | 0 | -1 | -1 | -1 | 1 | 1 | 1 | 0 | -1 | -1 | 0 | 1 | 1 |
| 33 | 0 | -1 | -1 | -1 | 0 | 1 | 1 | 1 | 0 | -1 | -1 | 0 | 1 | 1 | 1 | 0 | -1 | -1 | -1 |
| 34 | 0 | -1 | -1 | -1 | -1 | 0 | 1 | 1 | 1 | 0 | 0 | -1 | -1 | -1 | 0 | 1 | 1 | 1 | 1 |
| 35 | 0 | -1 | -1 | -1 | -1 | -1 | 0 | 0 | 1 | 1 | 1 | 1 | 0 | 0 | -1 | -1 | -1 | -1 | -1 |
| 36 | 0 | 0 | -1 | -1 | -1 | -1 | -1 | -1 | -1 | 0 | 0 | 1 | 1 | 1 |  | 1 | 1 | 1 | 0 |
| 37 | 0 | 0 | 0 | -1 | -1 | 1 | -1 | -1 | -1 | -1 | -1 | -1 | -1 | -1 | -1 | -1 | -1 | 0 | 0 |

Näherungen für $\cos_{n,i}$

Tabelle 2

| i | n | | | | | | | | | | | | | | | | | | |
|---|---|---|---|---|---|---|---|---|---|---|---|---|---|---|---|---|---|---|---|
| | 0 | 1 | 2 | 3 | 4 | 5 | 6 | 7 | 8 | 9 | 10 | 11 | 12 | 13 | 14 | 15 | 16 | 17 | 18 |
| 0 | 1 | 1 | 1 | 1 | 1 | 1 | 1 | 1 | 1 | 1 | 1 | 1 | 1 | 1 | 1 | 1 | 1 | 1 | 1 |
| 1 | 1 | 1 | 1 | 1 | 1 | 1 | 1 | 1 | 0 | 0 | 0 | 0 | 0 | -1 | -1 | -1 | -1 | -1 | 0 |
| 2 | 1 | 1 | 1 | 1 | 0 | 0 | -1 | -1 | -1 | -1 | -1 | -1 | -1 | -1 | 0 | 0 | 1 | 1 | 1 |
| 3 | 1 | 1 | 1 | 0 | 0 | -1 | -1 | -1 | -1 | 0 | 0 | 1 | 1 | 1 | 1 | 1 | 0 | -1 | -1 |
| 4 | 1 | 1 | 0 | -1 | -1 | -1 | -1 | 0 | 1 | 1 | 1 | 1 | 0 | -1 | -1 | -1 | -1 | 0 | 1 |
| 5 | 1 | 1 | 0 | -1 | -1 | -1 | 0 | 1 | 1 | 1 | -1 | -1 | -1 | 0 | 1 | 1 | 1 | 0 | -1 |
| 6 | 1 | 1 | -1 | -1 | -1 | 0 | 1 | 1 | 0 | -1 | -1 | 0 | 1 | 1 | 0 | -1 | 0 | -1 | 1 |
| 7 | 1 | 1 | -1 | -1 | 0 | 1 | 1 | 0 | -1 | -1 | 1 | 1 | 0 | -1 | -1 | 0 | 1 | 1 | -1 |
| 8 | 1 | 0 | -1 | -1 | 1 | 1 | 0 | -1 | 0 | 1 | 1 | -1 | -1 | 0 | 1 | 1 | -1 | -1 | 0 |
| 9 | 1 | 0 | -1 | 0 | 1 | 1 | -1 | -1 | 1 | 1 | -1 | -1 | 1 | 1 | -1 | -1 | 0 | 1 | 0 |
| 10 | 1 | 0 | -1 | 0 | 1 | -1 | -1 | 1 | 1 | -1 | 0 | 1 | 1 | -1 | 0 | 1 | 0 | -1 | 0 |
| 11 | 1 | 0 | -1 | 1 | 1 | -1 | 0 | 1 | -1 | -1 | 1 | 1 | -1 | 0 | 1 | -1 | -1 | 1 | 0 |
| 12 | 1 | -1 | -1 | 1 | 0 | -1 | 1 | 0 | -1 | 1 | 1 | -1 | 0 | 1 | -1 | 0 | 1 | -1 | -1 |
| 13 | 1 | -1 | 0 | 1 | -1 | 0 | 1 | -1 | 0 | 1 | -1 | 0 | 1 | -1 | 0 | 1 | -1 | 1 | 1 |
| 14 | 1 | -1 | 0 | 1 | -1 | 1 | 0 | -1 | 1 | -1 | -1 | 1 | -1 | 0 | 1 | -1 | 1 | 0 | -1 |
| 15 | 1 | -1 | 0 | 1 | -1 | 1 | -1 | 0 | 1 | -1 | 1 | -1 | 0 | 1 | -1 | 1 | -1 | 0 | 1 |
| 16 | 1 | -1 | 1 | 0 | -1 | 1 | -1 | 1 | -1 | 0 | 0 | -1 | 1 | -1 | 1 | -1 | 0 | 1 | -1 |
| 17 | 1 | -1 | 1 | -1 | 0 | 0 | 0 | 1 | -1 | 1 | -1 | 1 | -1 | 1 | 0 | 0 | 1 | -1 | 1 |
| 18 | 1 | -1 | 1 | -1 | 1 | -1 | 1 | -1 | 0 | 0 | 0 | 0 | -1 | 1 | -1 | 1 | -1 | 1 | -1 |
| 19 | 1 | -1 | 1 | -1 | 1 | -1 | 1 | -1 | 1 | -1 | 1 | -1 | 1 | -1 | 1 | -1 | 1 | -1 | 1 |
| 20 | 1 | -1 | 1 | -1 | 1 | -1 | 1 | -1 | 0 | 0 | 0 | 0 | 0 | 1 | -1 | 1 | -1 | 1 | 0 |
| 21 | 1 | -1 | 1 | -1 | 0 | 0 | -1 | 1 | -1 | 1 | -1 | 1 | -1 | 1 | 0 | 0 | 1 | -1 | 1 |
| 22 | 1 | -1 | 1 | 0 | 0 | 1 | -1 | 1 | -1 | 0 | 0 | -1 | 1 | -1 | 1 | -1 | 0 | 1 | -1 |
| 23 | 1 | -1 | 0 | 1 | -1 | 1 | -1 | 0 | 1 | -1 | 1 | -1 | 0 | 1 | -1 | 1 | -1 | 0 | 1 |
| 24 | 1 | -1 | 0 | 1 | -1 | 1 | 0 | -1 | 1 | -1 | -1 | 1 | -1 | 0 | 1 | -1 | 1 | 0 | -1 |
| 25 | 1 | -1 | -1 | 1 | -1 | 0 | 1 | -1 | 0 | 1 | -1 | 0 | 1 | -1 | 0 | 1 | 0 | 1 | 1 |
| 26 | 1 | -1 | -1 | 1 | 0 | -1 | 1 | 0 | -1 | 1 | 1 | -1 | 0 | 1 | -1 | 0 | 1 | -1 | -1 |
| 27 | 1 | 0 | -1 | 1 | 1 | -1 | 0 | 1 | 0 | -1 | 1 | 1 | -1 | 0 | 1 | -1 | -1 | 1 | 0 |
| 28 | 1 | 0 | -1 | 0 | 1 | -1 | -1 | 1 | 1 | -1 | -1 | 1 | 1 | -1 | -1 | 1 | 0 | -1 | 0 |
| 29 | 1 | 0 | -1 | 0 | 1 | 1 | -1 | -1 | 1 | 1 | 0 | -1 | 1 | 1 | 0 | -1 | 0 | 1 | 0 |
| 30 | 1 | 0 | -1 | -1 | 1 | 1 | 0 | -1 | -1 | 1 | 1 | -1 | -1 | 0 | 1 | 1 | -1 | -1 | 0 |
| 31 | 1 | 1 | -1 | -1 | 0 | 1 | 1 | 0 | -1 | -1 | 1 | 1 | 0 | -1 | -1 | 0 | 1 | 1 | -1 |
| 32 | 1 | 1 | 0 | -1 | -1 | 0 | 1 | 1 | 0 | -1 | -1 | 0 | 1 | 1 | 0 | -1 | -1 | -1 | 1 |

(fortgesetzt)

| i | n | | | | | | | | | | | | | | | | | | |
|---|---|---|---|---|---|---|---|---|---|---|---|---|---|---|---|---|---|---|---|
| | 0 | 1 | 2 | 3 | 4 | 5 | 6 | 7 | 8 | 9 | 10 | 11 | 12 | 13 | 14 | 15 | 16 | 17 | 18 |
| 33 | 1 | 1 | 0 | -1 | -1 | -1 | 0 | 1 | 1 | 1 | -1 | -1 | -1 | 0 | 1 | 1 | 1 | 0 | -1 |
| 34 | 1 | 1 | 0 | -1 | -1 | -1 | -1 | 0 | 1 | 1 | 1 | 1 | 0 | -1 | -1 | -1 | -1 | 0 | 1 |
| 35 | 1 | 1 | 1 | 0 | -1 | -1 | -1 | -1 | -1 | 0 | 0 | 1 | 1 | 1 | 1 | 1 | 0 | -1 | -1 |
| 36 | 1 | 1 | 1 | 1 | 0 | 0 | 0 | -1 | -1 | -1 | -1 | -1 | -1 | -1 | 0 | 0 | 1 | 1 | 1 |
| 37 | 1 | 1 | 1 | 1 | 1 | 1 | 1 | 1 | 0 | 0 | 0 | 0 | -1 | -1 | -1 | -1 | -1 | -1 | -1 |

**Patentansprüche**

**1.** Verfahren zur Berechnung der Herzratenvariabilität des menschlichen Herzens durch Auswertung des Frequenzspektrums der Herzrate zur Anwendung in einem EKG-Monitor mit folgenden Verfahrensschritten:

- Abtasten des vom EKG-Monitor empfangenen EKG-Signals während eines Abtastintervalls,
- Ermittlung einer Reihe diskreter, für die Herzratenvariabilität repräsentativer Meßwerte aus den abgetasteten EKG-Signalen, und
- Bestimmen der Herzatenvariabilität durch Auswerten dieser Meßwerte auf der Basis der Fourier-Transformation, wobei das Frequenzspektrum der Meßwerte aus den Fourier-Koeffizienten der Fourier-Transformation berechnet wird, und wobei die Fourier-Koeffizienten wiederum aus einer Kombination der Meßwerte mit den sinus- und kosinusförmigen Fourier-Vektoren der Fourier-Transformation berechnet werden, wobei die Fourier-Vektoren jeweils in Form einer Reihe diskreter Vektor-Zahlenwerte in die Berechnung einbezogen werden, **gekennzeichnet durch** folgende Verfahrensschritte:
- Ersetzen der Vektor-Zahlenwerte der Fourier-Vektoren für die Berechnung des Frequenzspektrums der abgetasteten Meßwerte **durch** ganzzahlige Vektor-Näherungswerte mit den Werten -1, 0 und + 1, wobei die Vektor-Näherungswerte in Abhängigkeit des tatsächlichen Vektor-Zahlenwertes wie folgt gesetzt werden:

- -0,33 < Vektor-Zahlenwert < +0,33: Vektor-Näherungswert = 0
- Vektor-Zahlenwert < -0,33: Vektor-Nähemngswert = -1
- Vektor-Zahlenwert > +0,33: Vektor-Näherungswert = + 1.

- Verwendung der ganzzahligen Vektor-Näherungswerte anstelle der sinus- und kosinusförmigen Feurier-Vektoren für die Berechnung des Frequenzspektrums

**2.** Verfahren nach Anspruch 1, **dadurch gekennzeichnet, daß** die Zuordnung der Näherungswerte zu den tatsächlichen Vektor-Zahlenwerten tabellarisch gespeichert im EKG-Monitor abgelegt sind.

**3.** Verfahren nach Anspruch 1, **dadurch gekennzeichnet, daß** die Zuordnung der Näherungswerte durch Berechnung der Punkte erfolgt, bei denen sich die Vektor-Näherungswerte ändern.

**4.** Verfahren nach einem der Ansprüche 1 bis 3, **dadurch gekennzeichnet, daß** die Summe der Vektor-Näherungswerte für jeden Fourier-Vektor gebildet und im Falle einer Abweichung vom Wert 0 mindestens ein Näherungswert korrigiert wird, bis der Summenwert 0 beträgt.

**5.** EKG-Monitor mit einem Betriebsprogramm **dadurch gekennzeichnet, daß** das Betriebsprogramm ein Berechnungsverfahren nach einem der Ansprüche 1 bis 4 implementiert.

**6.** EKG-Monitor nach Anspruch 5, **dadurch gekennzeichnet, daß** er in den menschlichen Körper implantierbar ist.

**7.** EKG-Monitor nach Anspruch 5 oder 6, **dadurch gekennzeichnet, daß** er in einen Herzschrittmacher integriert ist.

**Claims**

**1.** A method for calculating the heart rate variability of the human heart by evaluating the frequency spectrum of the heart rate for use in an ECG monitor with the following process steps:

- Scanning of the ECG signal received by the EKG monitor during a scanning interval,
- Determination of a number of discrete measured values representative of the heart rate variability from the scanned ECG signals and
- Determination of the heart rate variability by evaluating these measured values on the basis of the Fourier transform, wherein the frequency spectrum of the measured values is calculated from the Fourier coefficients of the Fourier transform, and wherein the Fourier coefficients in turn are calculated from a combination of the measured values with the sinusoidal and cosinusoidal Fourier vectors of the Fourier transform, wherein the Fourier vectors are respectively incorporated into the calculation in the form of a number of discrete numerical vector values,

**characterized by** the following process steps:

- Replacement of the numerical vector values of the Fourier vectors for the calculation of the frequency spectrum of the scanned measured values by integral approximate vector values with the values -1, 0 and +1, wherein the approximate vector values are set as follows depending on the actual numerical vector value:

- -0.33 < numerical vector value < +0.33:     Approximate vector value = 0
- Numerical vector valuer < -0.33:     Approximate vector value = -1
- Numerical vector value > +0.33:     Approximate vector value = +1

- Use of the integral approximate vector values instead of the sinusoidal and cosinusoidal Fourier vectors for the calculation of the frequency spectrum.

**2.** The method according to claim 1, **characterized in that** the assignment of the approximate values to the actual numerical vector values are stored in table form in the ECG monitor.

**3.** The method according to claim 1, **characterized in that** the assignment of the approximate values is carried out by calculating the points at which the approximate vector values change.

**4.** The method according to one of claims 1 through 3, **characterized in that** the sum of the approximate vector values for each Fourier vector is formed and in the event of a deviation from the value 0 at least one approximate value is corrected, until the sum value is 0.

**5.** An ECG monitor with an operating program **characterized in that** the operating program implements a calculation method according to one of claims 1 through 4.

**6.** The ECG monitor according to claim 5, **characterized in that** it can be implanted in the human body.

**7.** The ECG monitor according to claim 5 or 6, **characterized in that** it is integrated into a cardiac pacemaker.

**Revendications**

**1.** Procédé pour le calcul de la variabilité du rythme cardiaque du coeur humain, par évaluation du spectre de fréquences du rythme cardiaque, pour une utilisation dans un moniteur d'ECG, avec les étapes de procédé suivantes :

- détection d'un signal d'ECG reçu du moniteur d'ECG, en l'espace d'un intervalle de détection,
- détermination d'une série de valeurs de mesure représentatives de la variabilité du rythme cardiaque, à partir des signaux d'ECG détectés, et
- détermination de la variabilité du rythme cardiaque par évaluation de ces valeurs de mesure sur la base de la transformation de Fourier, où le spectre de fréquences des valeurs de mesure est calculé à partir des coefficients de Fourier de la transformation de Fourier, et où les coefficients de Fourier sont quant à eux calculés à partir de la combinaison des valeurs de mesure avec les vecteurs de Fourier en forme de sinus et cosinus de la transformation de Fourier, les vecteurs de Fourier étant respectivement inclus dans le calcul sous la forme d'une série de valeurs numériques discrètes de vecteur,

**caractérisé par** les étapes de procédé suivantes :

- remplacement des valeurs numériques de vecteur des vecteurs de Fourier pour le calcul du spectre de fréquences des valeurs de mesure détectées par des valeurs approchées entières de vecteur, avec les valeurs -1, 0 et +1, où les valeurs approchées sont fixées de la manière suivante, en fonction de la valeur numérique de vecteur réelle :

- -0,33 < valeur numérique de vecteur < +0,33 :
valeur approchée de vecteur = 0
- valeur numérique de vecteur < -0,33 :
valeur approchée de vecteur = -1
- valeur numérique de vecteur > + 0,33 :
valeur approchée de vecteur = +1.

- utilisation des valeurs approchées entières de vecteur à la place des vecteurs de Fourier en forme de sinus et cosinus pour le calcul du spectre de fréquences.

2. Procédé selon la revendication 1, **caractérisé en ce que** l'affectation des valeurs approchées aux valeurs numérique de vecteur réelles est enregistrée sous forme de tableau dans le moniteur d'ECG.

3. Procédé selon la revendication 1, **caractérisé en ce que** l'affectation des valeurs approchées est effectuée par calcul des points auxquels se modifient les valeurs approchées de vecteur.

4. Procédé selon l'une des revendications 1 à 3, **caractérisé en ce que** la somme des valeurs approchées de vecteur est formée pour chaque vecteur Fourier, et au moins une valeur approchée est corrigée en cas d'écart par rapport à la valeur 0, jusqu'à ce que la somme soit à 0.

5. Moniteur d'ECG avec un logiciel d'exploitation, **caractérisé en ce que** le logiciel d'exploitation exécute un procédé de calcul selon l'une des revendications 1 à 4.

6. Moniteur d'ECG selon la revendication 5, **caractérisé en ce qu'**il peut être implanté dans le corps humain.

7. Moniteur d'ECG selon la revendication 5 ou 6, **caractérisé en ce qu'**il est intégré dans un stimulateur cardiaque.

Fig. 1

Fig. 2

**Fig. 3**

**Fig. 4**

**Fig. 5**

**EP 1 124 186 B1**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 5967995 A

- WO 8602250 A